# EUROPEAN PATENT SPECIFICATION

(11) **EP 3 448 238 B1**
(45) Date of publication and mention of the grant of the patent: **07.05.2025**
(21) Application number: 16900321.7
(22) Date of filing: 29.04.2016
(51) Int. Cl.: A61B 5/00, A61B 5/0205, G16H 10/60, G16H 40/63, A61B 5/024, A61B 5/08

(54) **PHYSIOLOGICAL MEASUREMENT PROCESSING**
VERARBEITUNG VON PHYSIOLOGISCHEN MESSUNGEN
TRAITEMENT DE MESURE PHYSIOLOGIQUE

(43) Date of publication of application: 06.03.2019
(73) Proprietor: Nokia Technologies Oy, 02610 Espoo (FI)
(72) Inventor: SAVOLAINEN, Teemu, 37120 Nokia (FI)
(74) Representative: Espatent Oy
(86) International application number: PCT/FI2016/050276
(87) International publication number: WO 2017/187005

(56) References cited:
- US-A1- 2006 200 007
- US-A1- 2008 300 572
- US-A1- 2010 286 490
- US-A1- 2012 078 064
- US-A1- 2014 047 316
- US-A1- 2015 066 538
- US-B1- 9 013 294

## Description

### TECHNICAL FIELD

The present application generally relates to physiological measurement processing.

### BACKGROUND

This section illustrates useful background information without admission of any technique described herein representative of the state of the art.

Patients with heart disease may be monitored to detect cardiac events with various means such as a worn pendant. If such events are detected, verbal verification is obtained to a question produced by speech synthesis. The verbal verification can be analyzed by speech recognition and used to prevent false alarms. In some cases, the medical condition of a patient is monitored with implantable medical devices to detect deviation from desired characteristics. If the monitoring indicates a severe condition, an alert may be generated, but in minor deviations, the patient may be queried about her symptoms for holistic diagnostic procedures. US 2014/047316 A1 discloses a method and system to create a personal priority graph. US 9 013 294 B1 discloses, according to its abstract, an alarm system technology, in which an alarm event is detected at a property monitored by an alarm system when the alarm system was set in an armed state. US 2006/200007 A1 discloses an automatic etiology sequencing system. US 2008/300572 A1 discloses a wireless monitor for a personal medical device system. US 2010/286490 A1 discloses an interactive patient monitoring system using speech recognition.

### SUMMARY

The scope of protection sought for various embodiments of the invention is set out by the independent claims. According to a first example aspect of the present invention, there is provided a method as defined by claim 14.

The annotation may be received by monitoring output of the person and identifying the annotation in the output of the person. The output of the person comprise any of speech; utterance; gesture; textual output; use of a key; and any combination thereof.

The customized event detection data may comprise an anomaly limit for a physiological parameter. The anomaly limit may be a maximum or a minimum. The physiological parameter may concern any of heart rate; blood pressure; blood sugar; respiration rate; respiration flow rate; skin color; shivering; blood oxygen; electrocardiography; body temperature; and facial movement. The customized event detection data for a person comprises any of age; weight; height; normal blood pressure; indication of one or more illnesses of the person; and maximum pulse of the person. The customized event detection data may comprise an anomaly pattern for a plurality of physiological parameters. The anomaly pattern may comprise a condition for a combination of thresholds.

The method may comprise responsive to a first condition supplementing the obtained physiological measurement data with one or more given physiological parameters. The first condition may comprise detecting a predetermined event. The customized event detection data may define the first condition.

The prioritizing may comprise using a machine learning process to determine estimated significance of the detected event. The prioritizing may combine the estimated significance of the detected event and the temporally associated annotation. The method may comprise classifying the detected events based on the combination of the estimated significance of the detected event and the temporally associated annotation.

The method may comprise responsive to detecting a predetermined event prompting the person to issue the annotation. The prompting of the person to issue the annotation may depend on the physiological measurement data and on the customized event detection data.

The method may comprise sending the physiological measurement data to a remote data processing system. The method may comprise sending to the remote data processing system an indication of the detected event. The indication of the detected event may comprise the time of the detected event. The indication of the detected event may comprise an indication of a type of the detected event. The method may comprise sending to the remote data processing system the annotation.

The method may comprise storing and batch sending the obtained physiological measurement data and plural received annotations obtained and received over a period of time. The method may comprise batch sending the obtained physiological measurement data and plural received annotations based on a predetermined schedule and / or when a given volume of data has been collected. The method may comprise batch sending the obtained physiological measurement data and plural received annotations on detecting a predetermined event. The method may comprise batch sending the obtained physiological measurement data and plural received annotations on gaining a given network access. The method may comprise batch sending the obtained physiological measurement data and plural received annotations on receiving a delivery request. The delivery request may be received from the person. The delivery request may be received from a source other than the person. The source other than the person may be the remote data processing system or a person thereof.

The method may comprise producing a list of the detected events and associated annotations. The list may be ordered by the prioritizing. The list may comprise hyperlinks to corresponding physiological measurement data sections.

The obtaining of the physiological measurement data may comprise receiving information from a sensor. The sensor may be configured to continually measure at least one physiological property of the person. The sensor may be worn by the person. The sensor may be implanted. The obtaining of the physiological measurement data may comprise receiving information from a plurality of sensors. The sensors may measure same or different physiological properties.

The detecting of the event may be performed by a local processing unit. The local processing unit may be worn by the person. The local processing unit may be implanted. The local processing unit may be a portable device. The local processing unit may be a mobile communication device such as a mobile phone.

The prioritizing of the detected event may be performed by the local processing unit. Alternatively, the prioritizing of the detected event may be performed by a remote data processing system.

The remote data processing system may comprise a data cloud hosted server. The remote data processing system may comprise a supervisor terminal. The supervisor terminal may be configured to indicate the detected event and the annotation to the supervisor.

The method may further comprise receiving the feedback from the supervisor. The feedback may be received from the supervisor terminal. The supervisor may be a medically trained person such as a doctor. Alternatively, the supervisor may be an artificial intelligence circuitry configured to evaluate the physiological measurements using the annotations.

According to a second example aspect of the present invention, there is provided an apparatus as defined by claim 1.

The apparatus may comprise a user interface configured to receive the annotation from the person. The user interface may comprise a speech recognition circuitry configured to recognize spoken annotations from the person. The user interface may comprise a speech synthesis circuitry configured to output information to the user by speech. The speech recognition circuitry may be at least partly formed using the at least one processor. The speech synthesis circuitry may be at least partly formed using the at least one processor. The user interface may comprise a key configured to receive an annotation. The user interface may be configured to indicate a context for receiving context-sensitively the annotation. The user interface may be configured to prompt the annotation by one or more questions. The annotation may comprise one or more parts provided by the person at one or more times.

According to a third example aspect of the present invention, there is provided a computer program comprising computer executable program code configured to execute any method of the first example aspect.

The computer program may be stored in a computer readable memory medium.

Any foregoing memory medium may comprise a digital data storage such as a data disc or diskette, optical storage, magnetic storage, holographic storage, opto-magnetic storage, phase-change memory, resistive random access memory, magnetic random access memory, solid-electrolyte memory, ferroelectric random access memory, organic memory or polymer memory. The memory medium may be formed into a device without other substantial functions than storing memory or it may be formed as part of a device with other functions, including but not limited to a memory of a computer, a chip set, and a sub assembly of an electronic device.

The apparatus may comprise a memory and a processor that are configured to cause the apparatus to perform the method of the first example aspect.

Different non-binding example aspects and embodiments of the present invention have been illustrated in the foregoing. The embodiments in the foregoing are used merely to explain selected aspects or steps that may be utilized in implementations of the present invention. Some embodiments may be presented only with reference to certain example aspects of the invention. It should be appreciated that corresponding embodiments may apply to other example aspects as well.

### BRIEF DESCRIPTION OF THE DRAWINGS

For a more complete understanding of example embodiments of the present invention, reference is now made to the following descriptions taken in connection with the accompanying drawings in which:
Fig. 1 shows an architectural drawing of a system of an example embodiment;
Figs. 2a and 2b show a flow chart of a various process steps that are implemented in some example embodiments;
Fig. 3 shows an example of a prioritizing look-up table of an example embodiment;
Fig. 4 shows an example of a an event and annotation table of an example embodiment;
Fig. 5 shows a graph illustrating the detection of event data according to an example embodiment;
Fig. 6 shows some alternative scenarios of event detection taking the annotations into account according to an example embodiment;
Fig. 7 shows a chart illustrating development of detected events in an example; and
Fig. 8 shows a block diagram of a local processing unit.

### DETAILED DESCRIPTON OF THE DRAWINGS

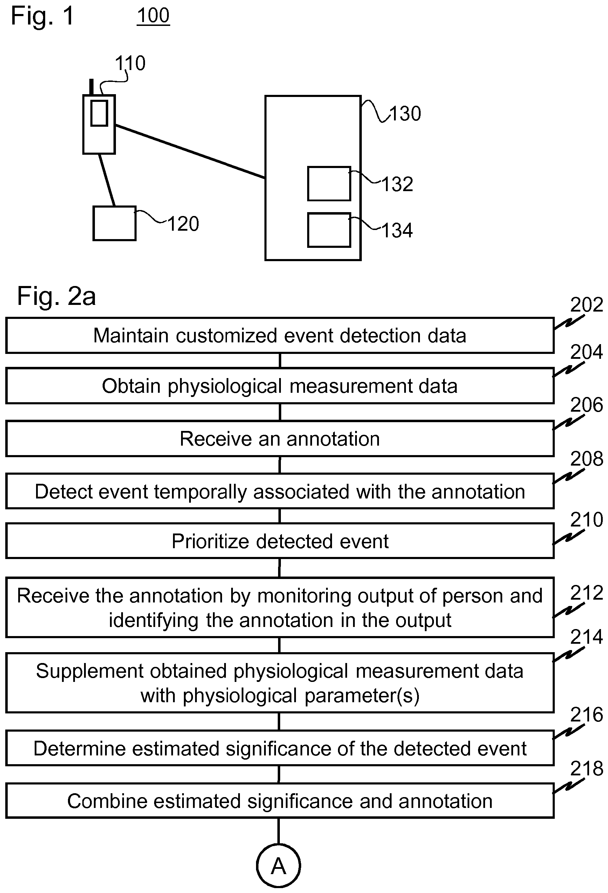

An example embodiment of the present invention and its potential advantages are understood by referring to Figs. 1 through 8 of the drawings. In this document, like reference signs denote like parts or steps.

Fig. 1 shows an architectural drawing of a system 100 of an example embodiment. The system comprises a local processing unit 110, one or more physiological measurement sensors 120 or biosensors in short (here only one is drawn in sake of simplicity), and a remote data processing system 130 comprising a plurality of supervisor terminals 132 and a database 134.

The local processing unit is in some implementations worn by the person, in some cases it can be implanted or a portable device or a mobile communication device such as a mobile phone. The local processing unit is in some embodiments integrated with at least one of the sensors 120.

In some embodiments, the remote data processing system 130 comprises a data cloud hosted server computer, a virtualized server computer, and / or a dedicated server computer.

The supervisor terminal can be used by a supervisor. The supervisor is, for example, a medically trained person such as a doctor or an artificial intelligence circuitry configured to evaluate the physiological measurements using the annotations.

Fig. 1 is simplified in that the remote data processing system 130 can typically operate with a large number of local processing units 110 and supervisor terminals 132.

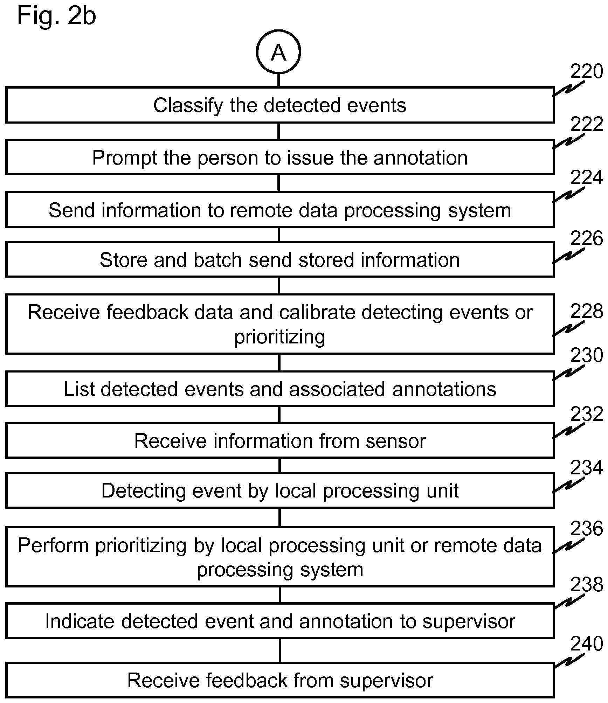

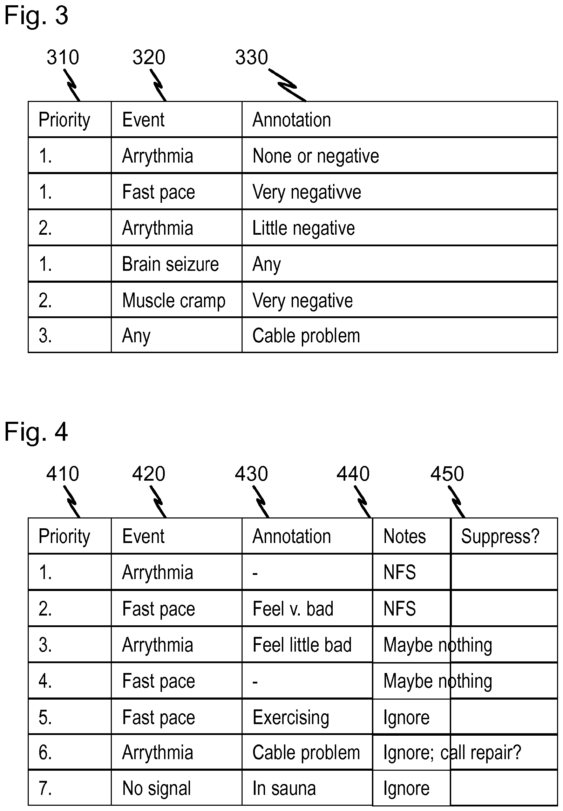

Figs. 2a and 2b show a flow chart of a various process steps that are implemented in some example embodiments. Notice that other than steps 202, 204, 206, 208, 210 and 228 that are a part of the claimed invention, not all the steps are necessarily taken, and some steps may be taken twice and also it is possible to further perform other steps in addition or instead of any of these steps. These steps include: 202 maintaining customized event detection data for a person; and automatically:
204 obtaining physiological measurement data indicative of physiological status of the person;
206 receiving an annotation from the person (in some embodiments, annotations can additionally be automatically created);
208 detecting an event that is temporally associated with the annotation using the physiological measurement data and the event detection data; and
210 prioritizing the detected event using the temporally associated annotation.
212 performing the receiving of the annotation by monitoring output of the user and identifying the annotation in the output of the user, the output of the user possibly comprising any of speech; utterance; gesture; textual output; use of a key; and any combination thereof, for example;
214 responsive to a first condition supplementing the obtained physiological measurement data with one or more given physiological parameters. In some embodiments, the first condition comprises detecting a predetermined event. The customized event detection data may define the first condition. For example, in case of heart patients, on meeting the first condition, an intrusive blood measurement may be made if the pulse exceeds a set threshold for the person in question.
216 Using a machine learning process to determine estimated significance of the detected event. For example, the limits of normal heart rate can be detected particularly using the annotations to verify that that peaks are exercise related or potentially interrelated patterns can be detected.
218 Combining, in the prioritizing, the estimated significance of the detected event and the temporally associated annotation. The combining of the estimated significance and the associated annotation enables highlighting potentially relevant event information for a supervisor such as a doctor very efficiently out of even large masses of physiological measurement data.
220 Classifying the detected events based on the combination of the estimated significance of the detected event and the temporally associated annotation.
222 Responsive to detecting a predetermined event prompting the person to issue the annotation. In some embodiments, the annotation can thus be enquired. For example, sometimes high pulse can be caused by emotional stimulus and the person facing such a situation might easily forget to provide annotations by speech, for example, of his or her own initiative. Moreover, the prompting can enable potentially identifying temporary loss of consciousness that could coincide with some unusual physiological changes that could appear in the measurements of the biosensor(s). The prompting can be implemented to take place depending on the physiological measurement data and on the customized event detection data for example such that persons with earlier heart attacks are easier prompted to annotate biosensor measurement changes that otherwise might not need further attention. The prompting can be made to direct the annotation to potentially useful information such as whether a person having a serious disease has taken the prescribed medication and whether she experiences symptoms that are commonly related to a disease that should be rapidly identified.
224 Sending information to a remote data processing system. The information comprises any of an indication of the detected event; the time of the detected event; an indication of a type of the detected event; and the annotation. By sending collected and/or derived information to the remote data processing system these data can be made available to a check by the supervisor. For example, some heart diseases are not always visible in the ECG graphs and modern technology may enable early detection of signs of a new stroke sufficiently in advance to take preventive action, if these signs are observed in time. Automatic diagnostics tools have been developed to help with this regard but their reliability and ability to compete with real doctors may still be limited and there may be liability reasons, for example, that inhibit the use of such tools. Automatic obtaining of physiological measurement data and event processing with reporting to the remote processing system may yet enable fast informing of a qualified supervisor of potentially relevant events. The diagnostic work can be left for such a professional or perhaps be performed by an artificial intelligence circuitry configured to perform the work of such a professional.
226 Storing and batch sending stored information. The stored information comprises any of the obtained physiological measurement data; received annotations obtained and received over a period of time; obtained physiological measurement data. The batch sending is timed in some embodiments based on a predetermined schedule. Additionally, or alternatively, the batch sending can be performed when a given volume of data has been gathered. The batch sending can be alternatively made only or additionally on any of: detecting a predetermined event; gaining a given network access; and receiving a delivery request. For example, the batch sending can be normally effected when a free network connection is available, unless there is detected an event that meets given conditions or a request is made by someone.
228 Receiving feedback data concerning the detecting of the event or the prioritizing of the detected events and calibrating the detecting of the event or the prioritizing of the detected events, respectively. The calibrating involves adjusting the customized event detection data.
230 Producing a list of the detected events and associated annotations. The list is ordered in some embodiments by the prioritizing, for example, and the list optionally comprises hyperlinks to corresponding physiological measurement data sections.
232 Receiving information from a sensor when obtaining the physiological measurement data, wherein the sensor is in some embodiments configured to continually measure at least one physiological property of the person. In some embodiments the sensor is worn by the person. In some cases, the sensor can be implanted. For example, a blood flow sensor could be implanted whereas a sweating sensor could be implemented with an on-the-skin sensor. Different sensors can be used in different embodiments without limitation to their type and implementation. For example, an artificial heart valve can be furnished with a sensor capable of wirelessly issuing (with RFID, for example) indications of the pulse or blood flow or the person could simply wear on her wrist a watch equipped with one or more sensors such as pulse and blood oxygen measurements.
234 Performing the detecting of the event by a local processing unit. The detecting of the event can be implemented in any suitable technique accounting for the nature of the sensor data and the nature of the event. For example, anomalies in pulse can be found by simply comparing the measured pulse to threshold limits, whereas anomalies in the ECG characteristics may require more complex processing such as determining the development curve of a signal or the mutual changes of measurements by different sensors.
236 Performing the prioritizing of the detected event by the local processing unit or by the remote data processing system 130. The annotations can be used in the detecting of the events so that some sensor data changes can be understood as very significant changes and others as simple malfunctions such as detachment of a sensor in accident or by the person's own choice. On the other hand, the annotations can be used alternatively or additionally in the prioritizing to weigh more or less some events based on the annotations. The prioritizing can be made using predetermined prioritizing criteria, which can be arranged using set functions or look-up tables, for example. Fig. 3 shows an example of a prioritizing look-up table that exemplifies preset priority values 310 for given combinations of detected most likely events 320 and the annotations 330 given by the person or obtained otherwise (e.g. by cable condition measurement).
238 Indicating the detected event and the annotation to the supervisor by the supervisor terminal. This can be performed, for example, by displaying a table or chart such as that shown in Fig. 4. The table shown in Fig. 4 comprises set priorities 410 based on the events 420 and annotations 430 and notes 440 recorded by the supervisor or fields in which such notes can be recorded if none present yet, and an optional suppress box 450 in which it can be defined that no further corresponding events should be reported at least in the presently used priority. In one embodiment, the suppressing results in lowering the priority of such events in future reports by one step, for example.
240 Receiving the feedback from the supervisor, using the supervisor terminal for example. This can be performed on the supervisor terminal by filling in particular feedback or notes fields in the table shown to the supervisor, for example, so as to enable simultaneous displaying of detected events, annotations of the person and the notes of the supervisor.

The customized event detection data comprise in an example embodiment an anomaly limit for a physiological parameter such as a maximum or a minimum. The physiological parameter concerns any of heart rate; blood pressure; blood sugar; respiration rate; respiration flow rate; skin color; shivering; blood oxygen; electrocardiography; body temperature; and facial movement, for example. The customized event detection data for a person comprise, for example, any of age; weight; height; normal blood pressure; indication of one or more illnesses of the person; and maximum pulse of the person. In some embodiments, the customized event detection data comprises an anomaly pattern for a plurality of physiological parameters. In an example embodiment, the anomaly pattern comprises a condition for a combination of thresholds.

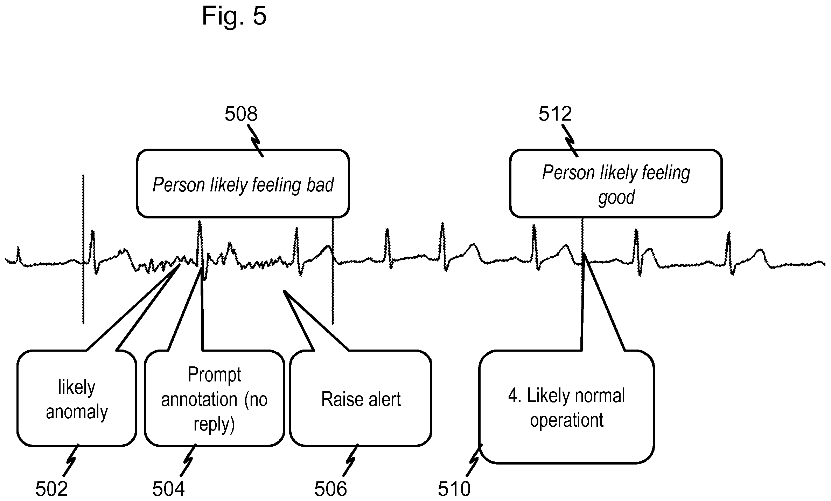

Fig. 5 illustrates the detection of event data by showing a graph and how events are detected and annotations given by the person. First, during a period when a person is likely feeling bad 508, a likely anomaly is detected, 502. In the absence of an unsolicited annotation, the person is prompted 504 to tell how she feels, but no response is received. Hence, an alert is raised 506. Then, during another period, the person is likely feeling good 512. Likely normal operation is detected 510. In some embodiments, then a supplemental report can be sent to the remote data processing system 130 or the earlier sent information may be corrected by clearing the alert, for example.

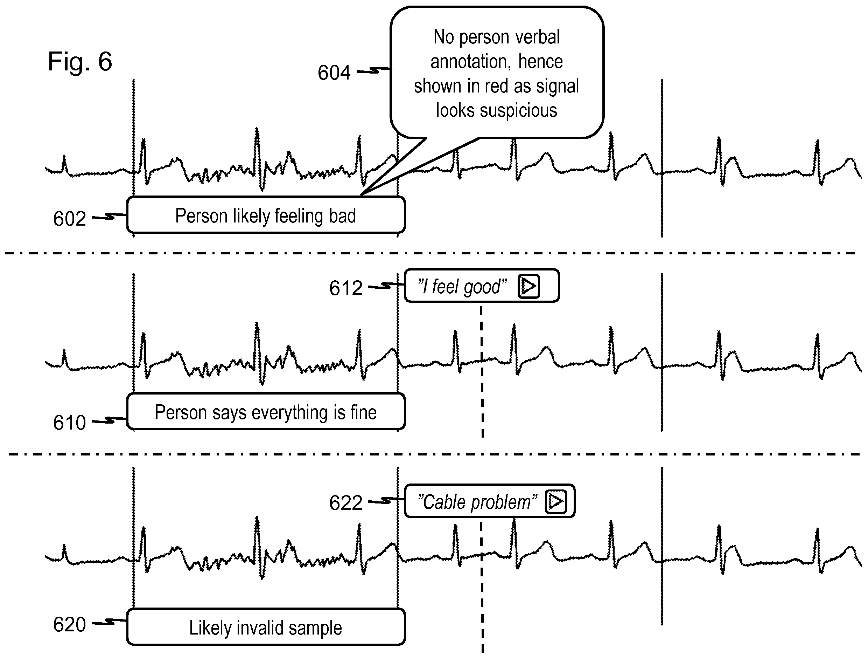

Fig. 6 shows some alternative scenarios of event detection taking the annotations into account. First, it is detected that the person is likely to feel bad by monitoring the sensor data, 602. No annotation is received from the person and the event is thus assigned a high priority as apparently suspicious, 604. Next, a similar sensor data is received in 610, but the person annotates that she feels good, 612. Hence, no action appears necessary and the event is classified to some intermediate priority level. Finally, a malfunction situation is presented, 620. Here, the person annotates that there was a cable problem, 622, and the event is classified as a technical problem.

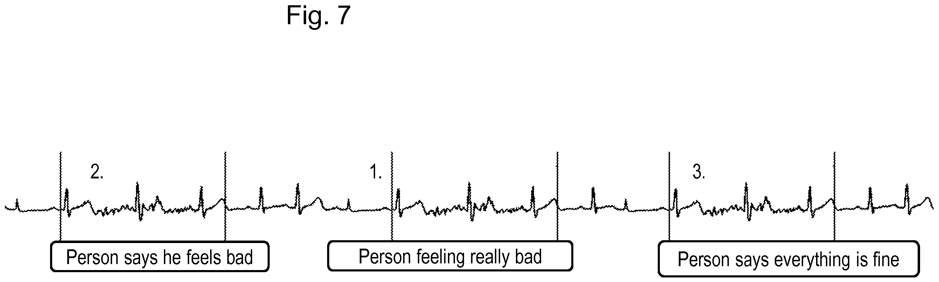

Fig. 7 shows an example of possible development of detected events, annotations and determined priorities formed by combining the detected events and annotations. In the case of Fig. 7 all the detected events are measurement-wise equal i.e. the measured signal is the same in each, hence prioritization of events is effectively determined based on annotations.

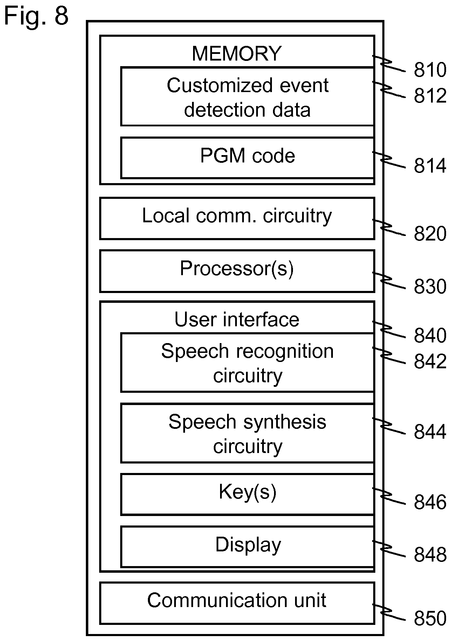

Fig. 8 shows a block diagram of the local processing unit 110 comprising: a memory 810 configured to maintain customized event detection data 812 for a person; a local communication circuitry 820 configured to obtain physiological measurement data indicative of physiological status of the person; at least one processor 830 configure to automatically perform: obtaining physiological measurement data indicative of physiological status of the person; receiving an annotation from the person; detecting an event that is temporally associated with the annotation using the physiological measurement data and the event detection data; and prioritizing the detected event using the temporally associated annotation.

The memory 810 can be used to store computer software such as executable program code 814 or instructions executing which the at least one processor may control operations of the local processing unit 110.

The local processing unit 110 of Fig. 8 further comprises a user interface 840 configured to receive the annotation from the person. The user interface of Fig. 8 comprises a speech recognition circuitry 842 configured to recognize spoken annotations from the person and a speech synthesis circuitry 844 configured to output information to the user (i.e. person) by speech. Either or both the speech recognition circuitry 842 and the speech synthesis circuitry 844 can be at least partly implemented using the at least one processor 830 or remote processing equipment. For example, speech of the person is recorded in one example embodiment and sent as such or with some pre-processing to a network-based processing function (e.g. a cloud-based server). Speech synthesis is at least partly distributed a function in one example embodiment so that the speech is at least partly generated in an external processing function and therefrom transferred to the local processing unit 110 for output to the person. The user interface of Fig. 8 further comprises a key 846 configured to receive an annotation, such as an emergency button and a display 848 for displaying information. The user interface can be configured to indicate a context for receiving context-sensitively the annotation under control of the at least one processor 830, for example. The user interface can configured to prompt the annotation by one or more specifying questions. The annotation may comprise one or more parts provided by the person at one or more times. The local processing unit 110 of Fig. 8 further comprises a communication unit 850 for communicating with the remote data processing system 130. The communication unit 850 comprises, for example, a local area network (LAN) port; a wireless local area network (WLAN) unit; a cellular data communication unit; or satellite data communication unit. The at least one processor 830 comprises, for example, any one or more of: a master control unit (MCU); a microprocessor; a digital signal processor (DSP); an application specific integrated circuit (ASIC); a field programmable gate array; and a microcontroller.

Without in any way limiting the scope, interpretation, or application of the claims appearing below, a technical effect of one or more of the example embodiments disclosed herein is that large amount of sensor data can be processed to identify potentially relevant events taking into account feedback of the person being measured and the measurement data can be appropriately prioritized for subsequent verification by a supervisor. Another technical effect of one or more of the example embodiments disclosed herein is that delivery of irrelevant alerts can be inhibited by receiving and processing annotations of the person.

Embodiments of the present invention may be implemented in software, hardware, application logic or a combination of software, hardware and application logic. The software, application logic and/or hardware may reside on the local processing unit 110, the remote data processing system 130 or both. If desired, part of the software, application logic and/or hardware may reside on the local processing unit 110, and a part of the software, application logic and/or hardware may reside on the remote data processing system 130. In an example embodiment, the application logic, software or an instruction set is maintained on any one of various conventional computer-readable media. In the context of this document, a "computer-readable medium" may be any non-transitory media or means that can contain, store, communicate, propagate or transport the instructions for use by or in connection with an instruction execution system, apparatus, or device, such as a computer, with one example of a computer described and depicted in Fig. 8. A computer-readable medium may comprise a computer-readable storage medium that may be any media or means that can contain or store the instructions for use by or in connection with an instruction execution system, apparatus, or device, such as a computer.

If desired, the different functions discussed herein may be performed in a different order and/or concurrently with each other. Furthermore, if desired, one or more of the before-described functions may be optional or may be combined.

It is also noted herein that while the foregoing describes example embodiments of the invention, these descriptions should not be viewed in a limiting sense. Rather, there are several variations and modifications which may be made without departing from the scope of the present invention as defined in the appended claims.

## Claims

1. An apparatus comprising means for performing:
maintaining customized event detection data for a person, comprising any of age; weight; height; normal blood pressure; indication of one or more illnesses of the person; and maximum pulse of the person;
obtaining physiological measurement data indicative of physiological status of the person;
receiving an annotation from the person;
detecting an event that is temporally associated with the annotation using the physiological measurement data and the customized event detection data;
prioritizing the detected event using the temporally associated annotation; and
receiving feedback data concerning the detecting of the event or the prioritizing of the detected events and calibrating the detecting of the event or the prioritizing of the detected events, respectively;
wherein the calibrating comprises adjusting the customized event detection data.

2. The apparatus of claim 1, wherein the apparatus is configured to receive the annotation by monitoring output of the person and identifying the annotation in the output of the person.

3. The apparatus of claim 2, wherein the output of the person comprises any of speech and utterance.

4. The apparatus of any preceding claim, comprising a speech recognition circuitry configured to recognize spoken annotations from the person.

5. The apparatus of any preceding claim wherein the customized event detection data comprises an anomaly limit for a physiological parameter.

6. The apparatus of any preceding claim wherein the means are further configured to perform, responsive to a first condition, supplementing the obtained physiological measurement data with one or more given physiological parameters.

7. The apparatus of claim 6, wherein the first condition comprises that a predetermined event is detected.

8. The apparatus of claim 6 or 7, wherein the customized event detection data defines the first condition.

9. The apparatus of any preceding claim wherein the prioritizing comprises using a machine learning process to determine estimated significance of the detected event.

10. The apparatus of any preceding claim wherein the means are further configured to perform, responsive to detecting a predetermined event, prompting the person to issue the annotation.

11. The apparatus of any preceding claim wherein the means are further configured to perform sending to a remote data processing system at least one of: the physiological measurement data, an indication of the detected event, and the annotation.

12. The apparatus of any preceding claim wherein the obtaining of the physiological measurement data comprises receiving information from a sensor.

13. The apparatus of any preceding claim wherein the means comprises
at least one processor; and
at least one memory including computer program code, the at least one memory and computer program code configured to, with the at least one processor, cause the performance of the apparatus.

14. A computer-implemented method comprising:
maintaining customized event detection data for a person, comprising any of age; weight; height; normal blood pressure; indication of one or more illnesses of the person; and maximum pulse of the person; and automatically:
obtaining physiological measurement data indicative of physiological status of the person;
receiving an annotation from the person;
detecting an event that is temporally associated with the annotation using the physiological measurement data and the customized event detection data;
prioritizing the detected event using the temporally associated annotation; and
receiving feedback data concerning the detecting of the event or the prioritizing of the detected events and calibrating the detecting of the event or the prioritizing of the detected events, respectively;
wherein the calibrating comprises adjusting the customized event detection data.

15. A computer program comprising instructions for causing an apparatus comprising a processor and a memory to perform at least the following:
maintaining customized event detection data for a person, comprising any of age; weight; height; normal blood pressure; indication of one or more illnesses of the person; and maximum pulse of the person;
obtaining physiological measurement data indicative of physiological status of the person;
receiving an annotation from the person;
detecting an event that is temporally associated with the annotation using the physiological measurement data and the customized event detection data;
prioritizing the detected event using the temporally associated annotation; and
receiving feedback data concerning the detecting of the event or the prioritizing of the detected events and calibrating the detecting of the event or the prioritizing of the detected events, respectively;
wherein the calibrating comprises adjusting the customized event detection data.

## Patentansprüche

1. Vorrichtung, die Mittel zum Durchführen von Folgendem umfasst:
Pflegen von benutzerdefinierten Ereignisdetektionsdaten für eine Person, die eines von einem Alter; einem Gewicht; einer Größe; einem normalen Blutdruck; einer Anzeige von einer oder mehreren Krankheiten der Person; und einem maximalen Puls der Person umfassen;
Erhalten von physiologischen Messdaten, die einen physiologischen Status der Person anzeigen;
Empfangen einer Anmerkung von der Person;
Detektieren eines Ereignisses, das zeitlich mit der Anmerkung verknüpft ist, unter Verwendung der physiologischen Messdaten und der benutzerdefinierten Ereignisdetektionsdaten;
Priorisieren des detektierten Ereignisses unter Verwendung der zeitlich verknüpften Anmerkung; und
Empfangen von Rückmeldungsdaten, die sich auf das Detektieren des Ereignisses oder das Priorisieren der detektierten Ereignisse beziehen, bzw. das Kalibrieren des Detektierens des Ereignisses oder des Priorisierens der detektierten Ereignisse beziehen;
wobei das Kalibrieren das Anpassen der benutzerdefinierten Ereignisdetektionsdaten umfasst.

2. Vorrichtung nach Anspruch 1, wobei die Vorrichtung dazu ausgelegt ist, die Anmerkung durch Überwachen einer Ausgabe der Person und Identifizieren der Anmerkung in der Ausgabe der Person zu empfangen.

3. Vorrichtung nach Anspruch 2, wobei die Ausgabe der Person eines von Sprache und einer Äußerung umfasst.

4. Vorrichtung nach einem der vorhergehenden Ansprüche, die eine Spracherkennungsschaltung umfasst, die dazu ausgelegt ist, gesprochene Anmerkungen von der Person zu erkennen.

5. Vorrichtung nach einem der vorhergehenden Ansprüche, wobei die benutzerdefinierten Ereignisdetektionsdaten eine Anomaliegrenze für einen physiologischen Parameter umfassen.

6. Vorrichtung nach einem der vorhergehenden Ansprüche, wobei die Mittel ferner dazu ausgelegt sind, in Reaktion auf eine erste Bedingung das Ergänzen der erhaltenen physiologischen Messdaten durch einen oder mehreren gegebenen physiologischen Parametern durchzuführen.

7. Vorrichtung nach Anspruch 6, wobei die erste Bedingung umfasst, dass ein vorbestimmtes Ereignis detektiert wird.

8. Vorrichtung nach Anspruch 6 oder 7, wobei die benutzerdefinierten Ereignisdetektionsdaten die erste Bedingung definieren.

9. Vorrichtung nach einem der vorhergehenden Ansprüche, wobei das Priorisieren das Verwenden eines Maschinenlernprozesses umfasst, um eine geschätzte Bedeutung des detektierten Ereignisses zu bestimmen.

10. Vorrichtung nach einem der vorhergehenden Ansprüche, wobei die Mittel ferner dazu ausgelegt sind, in Reaktion auf das Detektieren eines vorbestimmten Ereignisses das Auffordern der Person, die Anmerkung auszugeben, umfasst.

11. Vorrichtung nach einem der vorhergehenden Ansprüche, wobei die Mittel ferner dazu ausgelegt sind, das Senden von mindestens einem von Folgendem an ein entferntes Datenverarbeitungssystem durchzuführen: der physiologischen Messdaten, einer Anzeige des detektierten Ereignisses und der Anmerkung.

12. Vorrichtung nach einem der vorhergehenden Ansprüche, wobei das Erhalten der physiologischen Messdaten das Empfangen von Informationen von einem Sensor umfasst.

13. Vorrichtung nach einem der vorhergehenden Ansprüche, wobei die Mittel Folgendes umfassen
mindestens einen Prozessor; und
mindestens einen Speicher, der Computerprogrammcode beinhaltet, wobei der mindestens eine Speicher und der Computerprogrammcode dazu ausgelegt sind, mit dem mindestens einen Prozessor die Durchführung der Vorrichtung zu veranlassen.

14. Computerimplementiertes Verfahren, das Folgendes umfasst:
Pflegen von benutzerdefinierten Ereignisdetektionsdaten für eine Person, die eines von einem Alter; einem Gewicht; einer Größe; einem normalen Blutdruck; einer Anzeige von einer oder mehreren Krankheiten der Person; und einem maximalen Puls der Person umfassen; und automatisches:
Erhalten von physiologischen Messdaten, die einen physiologischen Status der Person anzeigen;
Empfangen einer Anmerkung von der Person;
Detektieren eines Ereignisses, das zeitlich mit der Anmerkung verknüpft ist, unter Verwendung der physiologischen Messdaten und der benutzerdefinierten Ereignisdetektionsdaten;
Priorisieren des detektierten Ereignisses unter Verwendung der zeitlich verknüpften Anmerkung; und
Empfangen von Rückmeldungsdaten, die sich auf das Detektieren des Ereignisses oder das Priorisieren der detektierten Ereignisse beziehen, bzw. das Kalibrieren des Detektierens des Ereignisses oder des Priorisierens der detektierten Ereignisse beziehen;
wobei das Kalibrieren das Anpassen der benutzerdefinierten Ereignisdetektionsdaten umfasst.

15. Computerprogramm, das Anweisungen zum Veranlassen einer Vorrichtung, die einen Prozessor und einen Speicher umfasst, mindestens Folgendes durchzuführen:
Pflegen von benutzerdefinierten Ereignisdetektionsdaten für eine Person, die eines von einem Alter; einem Gewicht; einer Größe; einem normalen Blutdruck; einer Anzeige von einer oder mehreren Krankheiten der Person; und einem maximalen Puls der Person umfassen;
Erhalten von physiologischen Messdaten, die einen physiologischen Status der Person anzeigen;
Empfangen einer Anmerkung von der Person;
Detektieren eines Ereignisses, das zeitlich mit der Anmerkung verknüpft ist, unter Verwendung der physiologischen Messdaten und der benutzerdefinierten Ereignisdetektionsdaten;
Priorisieren des detektierten Ereignisses unter Verwendung der zeitlich verknüpften Anmerkung; und
Empfangen von Rückmeldungsdaten, die sich auf das Detektieren des Ereignisses oder das Priorisieren der detektierten Ereignisse beziehen, bzw. das Kalibrieren des Detektierens des Ereignisses oder des Priorisierens der detektierten Ereignisse beziehen;
wobei das Kalibrieren das Anpassen der benutzerdefinierten Ereignisdetektionsdaten umfasst.

## Revendications

1. Appareil comprenant des moyens pour :
conserver des données de détection d'événement personnalisées pour une personne, comprenant l'un quelconque parmi l'âge ; le poids ; la taille ; une pression sanguine normale ; une indication d'une ou plusieurs maladies de la personne ; et le pouls maximum de la personne ;
obtenir des données de mesure physiologique indicatives d'un statut physiologique de la personne ;
recevoir une annotation de la personne ;
détecter un événement qui est associé temporellement à l'annotation en utilisant les données de mesure physiologique et les données de détection d'événement personnalisées ;
prioriser l'événement détecté en utilisant l'annotation associée temporellement ; et
recevoir des données de rétroaction concernant la détection de l'événement ou la priorisation des événements détectés et étalonner la détection de l'événement ou la priorisation des événements détectés, respectivement ;
dans lequel l'étalonnage comprend l'ajustement des données de détection d'événement personnalisées.

2. Appareil selon la revendication 1, dans lequel l'appareil est configuré pour recevoir l'annotation en surveillant la sortie de la personne et en identifiant l'annotation dans la sortie de la personne.

3. Appareil selon la revendication 2, dans lequel la sortie de la personne comprend une parole ou un énoncé.

4. Appareil selon l'une des revendications précédentes, comprenant un ensemble de circuits de reconnaissance de la parole configuré pour reconnaître des annotations parlées de la personne.

5. Appareil selon l'une des revendications précédentes dans lequel les données de détection d'événement personnalisées comprennent une limite d'anomalie pour un paramètre physiologique.

6. Appareil selon l'une des revendications précédentes dans lequel, en réponse à une première condition, les moyens sont en outre configurés pour compléter les données de mesure physiologique obtenues avec un ou plusieurs paramètres physiologiques donnés.

7. Appareil selon la revendication 6, dans lequel la première condition comprend la détection d'un événement prédéterminé.

8. Appareil selon la revendication 6 ou 7, dans lequel les données de détection d'événement personnalisées définissent la première condition.

9. Appareil selon l'une des revendications précédentes dans lequel la priorisation comprend l'utilisation d'un processus d'apprentissage machine pour déterminer une importance estimée de l'événement détecté.

10. Appareil selon l'une des revendications précédentes dans lequel, en réponse à la détection d'un événement prédéterminé, les moyens sont en outre configurés pour inviter la personne à émettre l'annotation.

11. Appareil selon l'une des revendications précédentes dans lequel les moyens sont en outre configurés pour envoyer à un système de traitement de données distant les données de mesure physiologique, une indication de l'événement détecté et/ou l'annotation.

12. Appareil selon l'une des revendications précédentes, dans lequel l'obtention des données de mesure physiologique comprend la réception d'informations provenant d'un capteur.

13. Appareil selon l'une des revendications précédentes dans lequel les moyens comprennent
au moins un processeur ; et
au moins une mémoire comportant un code de programme informatique, l'au moins une mémoire et le code de programme informatique étant configurés pour, avec l'au moins un processeur, permettre la performance de l'appareil.

14. Procédé mis en œuvre par ordinateur comprenant les étapes suivantes :
conserver des données de détection d'événement personnalisées pour une personne, comprenant l'un quelconque parmi l'âge ; le poids ; la taille ; une pression sanguine normale ; une indication d'une ou plusieurs maladies de la personne ; et le pouls maximum de la personne ; et automatiquement :
obtenir des données de mesure physiologique indicatives d'un statut physiologique de la personne ;
recevoir une annotation de la personne ;
détecter un événement qui est associé temporellement à l'annotation en utilisant les données de mesure physiologique et les données de détection d'événement personnalisées ;
prioriser l'événement détecté en utilisant l'annotation associée temporellement ; et
recevoir des données de rétroaction concernant la détection de l'événement ou la priorisation des événements détectés et étalonner la détection de l'événement ou la priorisation des événements détectés, respectivement ;
dans lequel l'étalonnage comprend l'ajustement des données de détection d'événement personnalisées.

15. Programme informatique comprenant des instructions pour amener un appareil comprenant un processeur et une mémoire à réaliser au moins ce qui suit :
conserver des données de détection d'événement personnalisées pour une personne, comprenant l'un quelconque parmi l'âge ; le poids ; la taille ; une pression sanguine normale ; une indication d'une ou plusieurs maladies de la personne ; et le pouls maximum de la personne ;
obtenir des données de mesure physiologique indicatives d'un statut physiologique de la personne ;
recevoir une annotation de la personne ;
détecter un événement qui est associé temporellement à l'annotation en utilisant les données de mesure physiologique et les données de détection d'événement personnalisées ;
prioriser l'événement détecté en utilisant l'annotation associée temporellement ; et
recevoir des données de rétroaction concernant la détection de l'événement ou la priorisation des événements détectés et étalonner la détection de l'événement ou la priorisation des événements détectés, respectivement ;
dans lequel l'étalonnage comprend l'ajustement des données de détection d'événement personnalisées.
